# EUROPEAN PATENT SPECIFICATION

(11) **EP 3 547 903 B1**
(45) Date of publication and mention of the grant of the patent: **06.10.2021**
(21) Application number: 17818642.5
(22) Date of filing: 27.11.2017
(51) Int. Cl.: A61B 5/00, A61H 31/00, G09B 23/28

(54) **COMPRESSION DEPTH CALCULATING DEVICE**
KOMPRESSIONSTIEFENBERECHNUNGSVORRICHTUNG
DISPOSITIF DE CALCUL DE PROFONDEUR DE COMPRESSION

(30) Priority: 29.11.2016 JP 2016231281
(43) Date of publication of application: 09.10.2019
(73) Proprietor: Nihon Kohden Corporation, Tokyo 161-8560 (JP)
(72) Inventor: INOUE Jumpei, Tokorozawa-shi Saitama 359-0037 (JP); WAKABAYASHI Tsutomu, Tokorozawa-shi Saitama 359-0037 (JP); AKIYAMA Naoto, Tokorozawa-shi Saitama 359-0037 (JP)
(74) Representative: Hoffmann Eitle
(86) International application number: PCT/JP2017/042390
(87) International publication number: WO 2018/101202

(56) References cited:
- EP-A1- 1 491 176
- EP-A2- 3 225 226
- US-A1- 2008 208 082
- US-A1- 2013 226 049
- US-A1- 2017 281 461

## Description

### Technical Field

The present invention relates to a compression depth calculating device.

### Background Art

CPR (CardioPulmonary Resuscitation) is an important manual operation in emergency medical care. Adequacy of the CPR may affect life and death of a rescuee (patient). When the CPR is performed, a rescuer compresses the sternum of the chest of the patient. Thus, the sternal compression can be performed in place of the patient's heart to circulate blood all over the patient's living body.

For example, according to "JRC Resuscitation Guideline 2015 (Japan Resuscitation Council)", it is recommended to perform manual sternal compression on an adult of normal physique to reach a depth of about 5 cm (or a depth of one third of a thickness of the chest in the case of a child) while avoiding excessive compression exceeding 6 cm.

It is necessary to make further studies about a relation between the compression depth and an external injury, and further about how the relation is affected by body or chest size, chest wall compliance, and a difference between adults and children. Further, it is also necessary to make further studies about a relation of interaction between a pace and the depth of the sternal compression.

In order to guide a rescuer during CPR, there have been developed apparatuses each placed between the chest of a rescuee and hands of the rescuer to assist sternal compression. Each of the apparatuses detects whether adequate force is applied to the chest with an adequately depth and at an adequate time interval, and gives appropriate notification ("force or compression depth is insufficient", "compression timing is too late", etc.) to the rescuer in accordance with the detection.

For example, a background-art apparatus has an acceleration sensor for calculation of a compression depth of sternal compression using a physical law that second-order integration of acceleration is a distance. However, the compression depth obtained from the second-order integration of the acceleration contains lots of error components due to unintentional vibration etc. In order to reduce the influence of the error components caused by the vibration etc. to thereby improve accuracy of the compression depth, a configuration which uses an average value of compression depths in a plurality of compression operations is therefore conceivable. In this configuration, however, a compression depth that is suddenly changed cannot be calculated accurately when such a sudden change in compression depth appears in one of the plural compression operations.

To solve this problem, a method for physically modeling the chest of a patient to calculate a compression depth has been proposed (see Patent Literature 1).

An alternative approach to the problem has been proposed by Patent Literature 2 which proposes to estimate the compression depth by combining the output of two sensors.

### Citation List

### Patent Literature

PTL 1: Japanese Patent No. 5508545

PTL 2: US 2013/226049

### Non Patent Literature

NPL 1: A.E. Tomlinson, et. al, "Compression force-depth relationship during out-of-hospital cardiopulmonary resuscitation", ELSEVIER 2006, pp 364-370.

### Summary of Invention

### Technical Problem

However, according to the technique in Patent Literature 1, accuracy in calculating the compression depth deteriorates when a compression displacement varies from one compression operation to another.

An object of the invention is to provide a compression depth calculating device capable of calculating a compression depth accurately even when a compression displacement varies from one compression operation to another.

### Solution to Problem

The compression depth calculating device according to the invention is defined by independent claim 1. Preferred embodiments are defined by the dependent claims.

According to the compression depth calculating device having the aforementioned configuration, the information corresponding to the magnitude of the compression of the compression operation which is acquired from the second sensor is inputted to the correlation between the compression depth calculated based on the second-order integration of the acceleration information acquired from the first sensor, and the information corresponding to the magnitude of the compression of the compression operation which has been acquired from the second sensor. With this configuration, since information which is acquired from the second sensor is applied to the correlation, a compression depth can be calculated accurately even when a sudden change in compression depth appears in one of compression operations.

Thus, according to the aforementioned configuration, the compression depth can be calculated accurately even when a compression displacement varies from one compression operation to another.

### Brief Description of Drawings

[fig.1]Fig. 1 is a sectional view of a measurement device of a cardiopulmonary resuscitation assisting apparatus provided with a compression depth calculating device according to an embodiment of the invention.
[fig.2]Fig. 2 is a block diagram of an overall configuration of the compression depth calculating device according to the embodiment.
[fig.3]Fig. 3 is a view showing the configuration of the measurement device etc.
[fig.4]Fig. 4 is a graph showing a relation between compression force and compression depth.
[fig.5]Fig. 5 is a graph showing relations between compression force and compression depth in different rescuees.
[fig.6]Fig. 6 is a graph showing a correlation between coil-to-coil distance and compression depth.
[fig.7]Fig. 7 is a graph showing a compression depth during a compression operation.
[fig.8]Fig. 8 is a schematic view showing an average correlation obtained from a plurality of correlations.
[fig.9]Fig. 9 is a graph showing a method of obtaining a compression depth using the average correlation.

### Description of Embodiments

An embodiment of a compression depth calculating device according to the invention will be described below by way of example with reference to the drawings. The invention is not limited to the illustrated examples and it is defined by the scope of the appended claims.

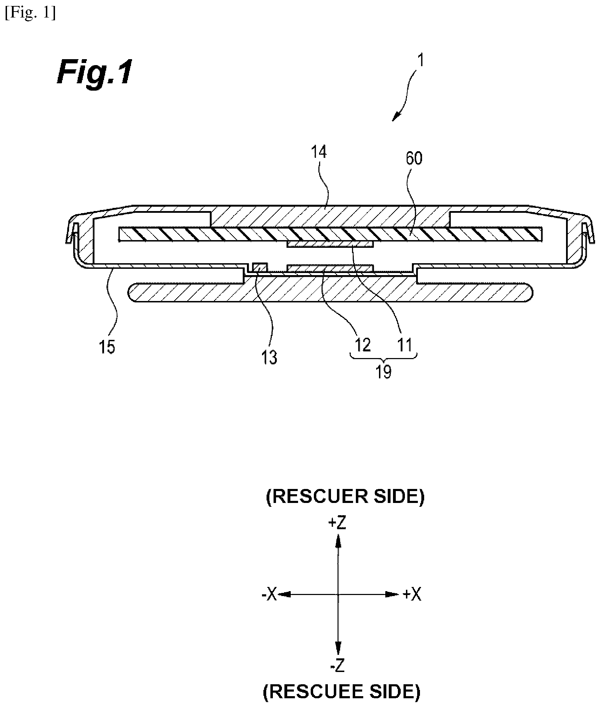

In Fig. 1, the reference sign 1 designates a measurement device of a cardiopulmonary resuscitation assisting apparatus. The measurement device 1 is a device which is placed between the chest (preferably right above the sternum) of a rescuee and hands of a rescuer to assist sternal compression. Incidentally, the rescuee is a concept including not only a human invalid (or also expressed as patient) but also a mannequin etc. That is, the cardiopulmonary resuscitation assisting apparatus may be used at a scene of real cardiopulmonary resuscitation or may be used at the time of cardiopulmonary resuscitation training. The cardiopulmonary resuscitation assisting apparatus detects compression depths and a number of times of sternal compression using displacements detected by a sensor between whenever the chest of the rescuee is pressed and whenever the chest of the rescuee is released from pressing. Therefore, the cardiopulmonary resuscitation assisting apparatus is required to have spring characteristics with which a displacement is generated whenever the chest of the rescuee is pressed.

A housing of the measurement device 1 of the cardiopulmonary resuscitation assisting apparatus includes a fixed section 15 and a movable section 14. The fixed section 15 and the movable section 14 are fitted into each other to thereby form the housing.

In the following description, directional axes (X axis, Z axis) are determined, as shown in Fig. 1. In addition, since a face of the cardiopulmonary resuscitation assisting apparatus in a positive direction of the Z axis (+Z direction) makes contact with the rescuer, the +Z direction will be also referred to as "rescuer side". Similarly, since a face of the cardiopulmonary resuscitation assisting apparatus in a negative direction of the Z axis (-Z direction) makes contact with the rescuee, the -Z direction will be also referred to as "rescuee side".

The measurement device 1 of the cardiopulmonary resuscitation assisting apparatus is mounted on the chest (preferably right above the sternum) of the rescuee with the fixed section 15 facing downward. The rescuer performs sternal compression to press a planar place of the movable section 14. When the rescuer performs the pressing, pressure is transmitted to the chest of the rescuee from a bottom direction of the fixed section 15. The rescuer who is mostly an adult fixes his/her hands to the measurement device 1 of the cardiopulmonary resuscitation assisting apparatus to perform pressing. Therefore, it is preferable that the measurement device 1 of the cardiopulmonary resuscitation assisting apparatus has a size fitted to the size of an adult's palm.

Here, the sternal compression will be described briefly. Adequacies in (1) the number of times, (2) depth (compression depth) and (3) returning of the sternal compression largely affect a rescue effect of the sternal compression. It has been considered that the number of times of the sternal compression is preferably at least about 100 to 120 times per minute. In addition, as to the compression depth of the sternal compression, it is recommended to manually perform the sternal compression on an adult of normal physique to reach a depth of about 5 cm (or a depth of one third of a thickness of the chest in the case of a child) while avoiding excessive compression exceeding 6 cm. When the compression depth is too small, a massage effect on the heart is insufficient. On the other hand, when the compression depth is too large, there is a fear that the sternum etc. may be broken. In addition, the chest is required to be decompressed sufficiently every time immediately after the compression is performed by manual operation of the rescuer. When the decompression is not sufficient, blood circulation becomes insufficient. The cardiopulmonary resuscitation assisting apparatus measures an actual compression depth and the number of times of compression (a compression rate), and compares these values with indices (5 to 6 cm, 100 to 120 times/minute).

The movable section 14 is a member to which pressure is directly applied by the manual operation of the rescuer, and which is a non-repulsive member (member having no spring characteristic). The movable section 14 is physically connected to the fixed section 15 and a printed board 60.

Various circuits and software for detecting and measuring strength of the sternal compression performed by the rescuer and a frequency of the sternal compression are mounted in the printed board 60.

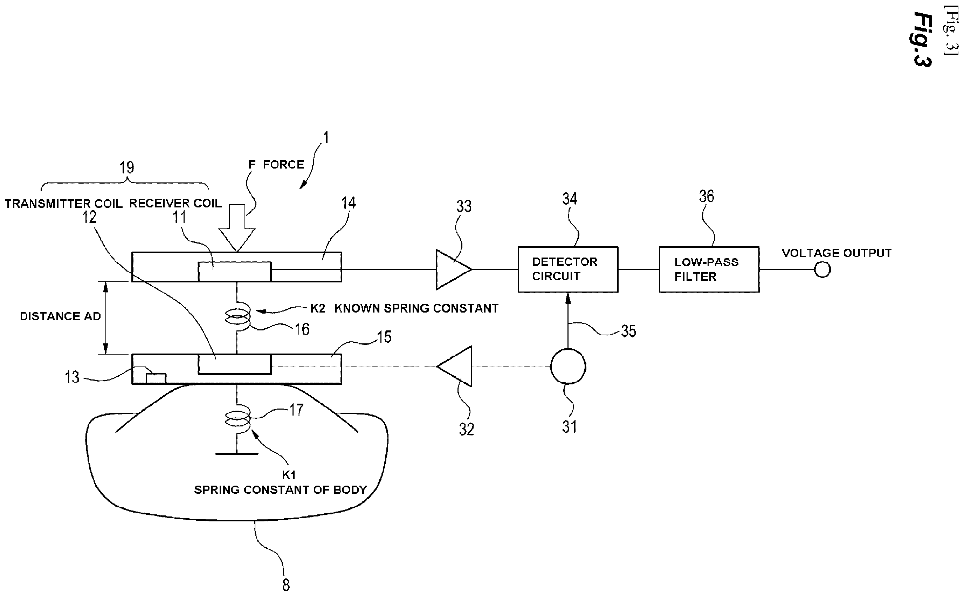

The fixed section 15 is a member (repulsive member) having spring characteristics. In other words, the fixed section 15 can be regarded as a configuration having a spring 16 (see Fig. 3) which is bent in the +Z direction (rescuer side) during pressing of the sternal compression and which is restored toward the -Z direction (rescuee side) during release of the pressing.

The measurement device 1 of the cardiopulmonary resuscitation assisting apparatus has a receiver coil (magnetic field detecting section) 11 which is provided in the movable section 14, and a transmitter coil (magnetic field generating section) 12 which is provided in the fixed section 15. In addition, the measurement device 1 of the cardiopulmonary resuscitation assisting apparatus has an acceleration sensor (first sensor) 13 which is provided in the fixed section 15.

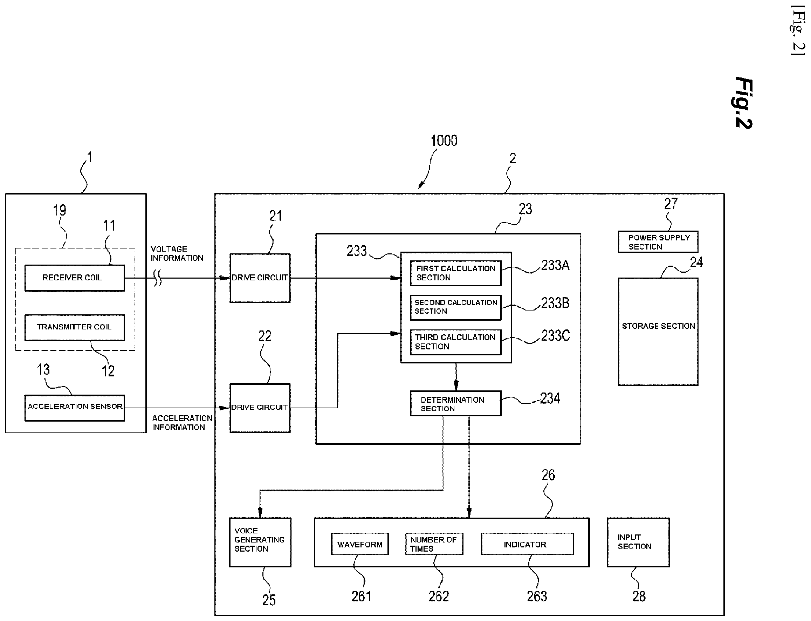

As shown in Fig. 2, the cardiopulmonary resuscitation assisting apparatus is provided with a compression depth calculating device 1000. The compression depth calculating device 1000 is configured to include the measurement device 1 and a calculation device 2.

As shown in Fig. 3, the measurement device 1 is configured to include the receiver coil 11 (magnetic field detecting section), the transmitter coil 12 (magnetic field generating section), the acceleration sensor 13, the movable section 14 and the fixed section 15. Here, the spring characteristics of the fixed section 15 are extracted as the spring 16 (elastic body) and clearly stated in the description. Incidentally, the receiver coil 11 and the transmitter coil 12 are combined to be referred to as a magnetic sensor 19 (second sensor).

The transmitter coil 12 and the acceleration sensor 13 are disposed in the fixed section 15. The fixed section 15 is fixed to a body B of the rescuee. For example, a method for disposing the body B on an anti-slipping sheet is considered as a fixation method. Here, the body B has spring characteristics and damper characteristics. Of them, the spring characteristics are dominant. Accordingly, the body B can be approximately regarded as a spring 17 having a spring constant K1. Incidentally, the spring constant K1 may be not lower than secondary.

The receiver coil 11 is disposed in the movable section 14 to be opposed to the transmitter coil 12. The spring 16 made of the fixed section 15 and having a spring constant K2 is disposed between the movable section 14 and the fixed section 15. Incidentally, the fixed section 15 serving as the spring 16 is selected so as to establish a relation K2>K1. Otherwise, when force F of compression is applied to the movable section 14, the spring 16 will contract up to a shortest length and a movable region will be hence limited. Therefore, a role as the magnetic sensor 19 will be spoiled. Incidentally, it is desirable to set a distance between the movable section 14 and the fixed section 15, for example, at about 5 mm.

Next, operations of the magnetic sensor 19 and peripheral components will be described with reference to Fig. 3. First, an AC (alternating current) oscillator 31 creates an AC voltage with a specific frequency (e.g. 20 kHz). The AC voltage is converted into an AC current with the specific frequency by an amplifier 32. The converted AC current flows into the transmitter coil 12. A magnetic field generated by the AC current flowing through the transmitter coil 12 generates an induced electromotive force in the receiver coil 11.

An AC current (having a frequency which is the same as the frequency of the AC voltage created by the AC oscillator 31) occurring in the receiver coil 11 due to the induced electromotive power is amplified by a preamplifier 33. A signal of the amplified AC current is inputted to a detector circuit 34. In the detector circuit 34, the signal of the amplified AC current is detected by the specific frequency created by the AC oscillator 31 or a frequency twice as high as the specific frequency. Therefore, an output of the AC oscillator 31 is introduced as a reference signal 35 into a reference signal input terminal of the detector circuit 34. Incidentally, when a full-wave rectifier circuit is used without using the detector circuit 34 and the reference signal 35, the measurement device 1 may be operated by the circuit. Thus, the size and price of the measurement device 1 can be reduced by the configuration of the full-wave rectifier circuit. After having passed through a low-pass filter 36, voltage information (output signal) from the detector circuit 34 (or the full-wave rectifier circuit) is introduced into a drive circuit 21 (see Fig. 2) of the calculation device 2.

As shown in Fig. 2, the calculation device 2 is a computer device which is configured to include the drive circuit 21, a drive circuit 22, a processing section 23, a storage section 24, a voice generating section 25, a display section 26, a power supply section 27, and an input section 28. In addition, the calculation device 2 may have a communication section for communicating with an external device.

The drive circuit 21 transmits, to the processing section 23, the voltage information received via the low-pass filter 36 (see Fig. 3) etc. from the receiver coil 11 of the measurement device 1.

The drive circuit 22 converts acceleration information received from the acceleration sensor 13 of the measurement device 1 into a voltage, and transmits the converted voltage to the processing section 23.

The processing section 23 is implemented, for example, by a CPU (Central Processing Unit). The processing section 23 is provided with a calculation section 233 and a determination section 234.

The calculation section 233 performs various calculations. The determination section 234 performs various determinations. The calculation section 233 is provided with a first calculation section 233A, a second calculation section 233B and a third calculation section 233C. The storage section 24 is a unit which stores various information. The storage section 24 is implemented, for example, by an RAM (Random Access Memory), an ROM (Read Only Memory), an HDD (Hard Disk Drive), or the like. The voice generating section 25 is a unit which generates voice. The voice generating section 25 is implemented, for example, by a speaker. The display section 26 is a unit which performs various displays. The display section 26 is implemented, for example, by an LCD (Liquid Crystal Display) or a CRT (Cathode Ray Tube) display. A waveform 261, a number of times 262, an indicator 263, etc. are displayed on the display section 26. The waveform 261 expresses a state of change of a compression depth with time. The number of times 262 expresses a number of times compression has been performed. The indicator 263 expresses a size of the compression depth. The power supply section 27 is a power supply unit in the calculation device 2. The input section 28 is a unit which is operated by a user in order to input various information. The input section 28 is implemented, for example, by a keyboard, a mouse, a switch, or the like.

During CPR held once, the rescuer performs compression operation, for example, at a frequency of 120 times per minute. On this occasion, it is preferable that the rescuer continuously gives, for example, a constant compression displacement of 5 cm or more to the chest of a patient in each compression operation. However, the compression operation repeated at a constant pace and with constant strength is a considerable load on the rescuer. For this reason, the compression displacement may vary from one compression operation to another. In addition, the rescuer may be taken over by another rescuer in the middle of the manual operation in order to disperse the load. Also in this case, the compression displacement may vary from one compression operation to another.

Thus, it can be fully predicted that the compression displacement may vary from one compression operation to another when the manual operation is actually performed. To solve this problem, there is an increasing demand for a device which can calculate a compression depth accurately even when the compression displacement varies from one compression operation to another.

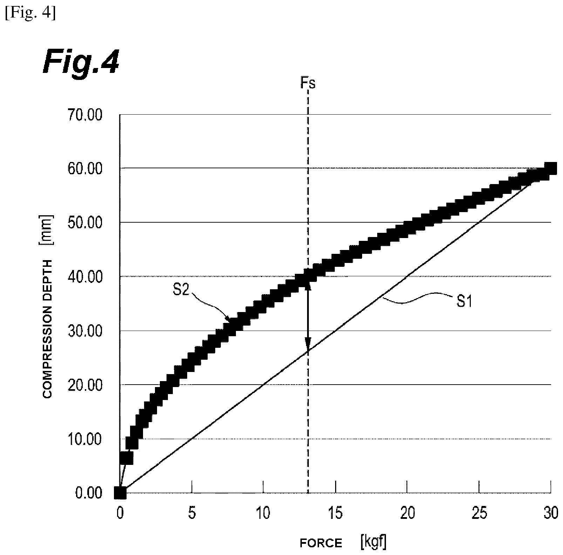

On the other hand, according to the background-art technique in Patent Literature 1, the chest of the rescuee is modeled linearly on the assumption that the spring constant of the chest of the rescuee is fixed. In the linear model, a compression depth can be calculated accurately when the compression depth in each compression operation falls into a fixed range. However, as shown in Fig. 4, in contrast to the linear model in which the compression depth is modeled linearly (as designated by a reference sign S1), an actual chest has a spring constant which can change in accordance with the compression depth to be non-linear (as designated by a reference sign S2). Accordingly, a relation between the compression depth and compression force should be modeled non-linearly.

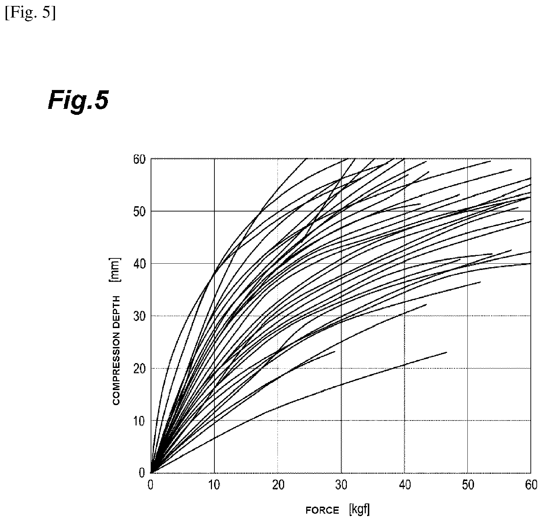

The present inventor has recognized that an influence of the linear model of a non-linear chest appears largely particularly in a case where the compression depth changes suddenly in one of plural compression operations, and the influence is not negligible. For example, assume that the compression force is weak (as designated by Fs in Fig. 4). In this case, a large deviation may occur in the compression depth between the linear model S1 and the non-linear model S2. In addition, due to individual differences in physique among patients, the relation between the change of the compression depth and the compression force varies from one patient to another, as shown in Fig. 5.

Therefore, the calculation section 233 of the compression depth calculating device 1000 according to the invention calculates the compression depth accurately even when a sudden change in compression depth appears in one of the compression operations.

A method using the calculation section 233 of the calculation device 2 of the compression depth calculating device 1000 to calculate the compression depth will be described below.

### (Extraction of Correlation)

In the calculation section 233, the first calculation section 233A applies second-order integration to acceleration acquired from the acceleration sensor 13 to calculate a compression depth Da. The first calculation section 233A obtains a correlation R between the compression depth Da which has been calculated based on the second-order integration of the acceleration information acquired from the acceleration sensor 13 and a coil-to-coil distance AD which is a distance between the receiver coil 11 and the transmitter coil 12 acquired from the magnetic sensor 19. The coil-to-coil distance AD is an example of information corresponding to a magnitude of compression.

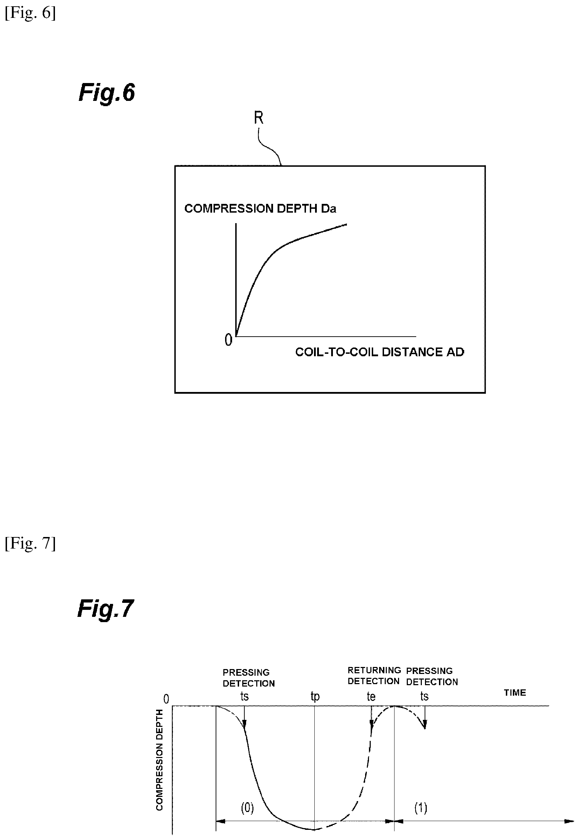

As shown in Fig. 6, the correlation R shows a change amount of the compression depth Da relative to the coil-to-coil distance AD, the compression depth Da being obtained by applying second-order integration to the acceleration acquired from the acceleration sensor 13. Since a spring constant of an actual chest changes in accordance with the compression depth, the spring constant of the actual chest may be non-linear. Incidentally, a spring constant of a linear spring used in a doll for practicing sternal compression may be linear.

Here, Fig. 7 shows a change of a compression depth of an actual chest when the chest of a rescuee is compressed. In the change of the compression depth, the first calculation section 233A obtains a correlation R between a pressing detection time (ts) and a compression depth peak time (tp) in a section between pressing detection (ts) and returning detection (te) determined based on a change of the coil-to-coil distance AD.

Incidentally, the correlation R is not always limited to a correlation which can be expressed by a mathematical expression. The correlation R is however a concept including data aggregate of a dispersion diagram in which a relation between data A and data B is plotted on two-dimensional coordinates, a table, etc. Incidentally, data may be interpolated between the plotted data by linear interpolation etc. or a least-squares method may be used on the collected data to mathematically obtain a relational expression as the correlation R.

### (Extraction of Average Correlation)

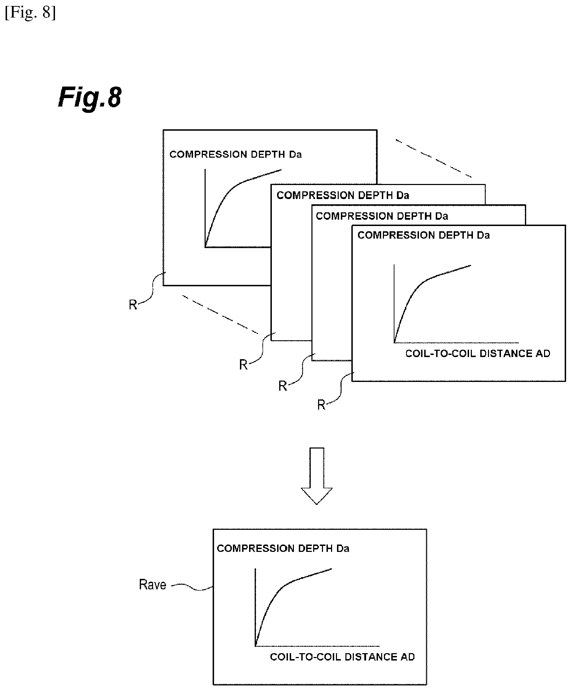

In the calculation section 233, the third calculation section 233C obtains an average correlation Rave using a plurality of (sixteen in this example) correlations R obtained in the first calculation section 233A, as shown in Fig. 8. The average correlation Rave is an average of the plurality of correlations R. Noise may be contained in a second-order integrated value of acceleration acquired from the acceleration sensor 13. However, when the average correlation Rave is obtained as the average of the plurality of correlations R, the influence of the noise can be reduced.

Here, the third calculation section 233C of the calculation section 233 always obtains an average correlation Rave using a predetermined number of (e.g. sixteen) correlations R immediately before a compression operation. When the number of correlations R immediately before the compression operation is smaller than the predetermined number in an early period after the compression start, the average correlation Rave is obtained using the plurality of correlations R smaller in number than the predetermined number.

### (Calculation of Compression Depth)

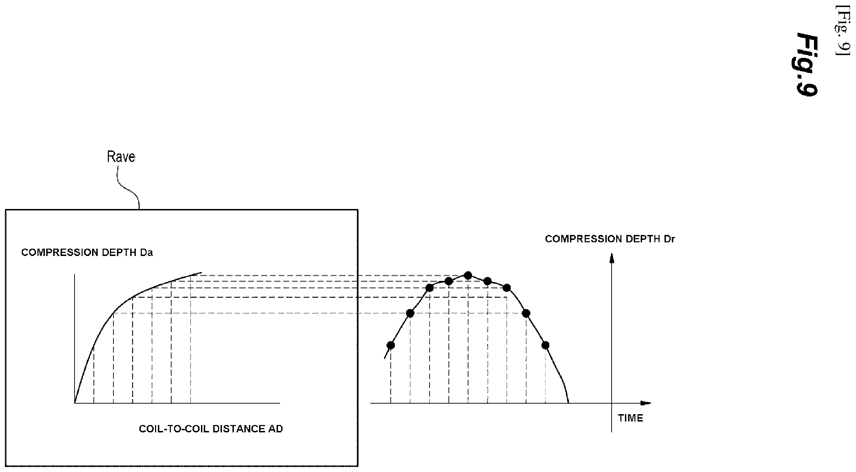

In the calculation section 233, the second calculation section 233B calculates a compression depth Dr of a compression operation performed after the average correlation Rave has been calculated, based on a coil-to-coil distance AD and the average correlation Rave when the compression operation is performed after the average correlation Rave has been calculated, as shown in Fig. 9. The coil-to-coil distance AD is a distance between the receiver coil 11 and the transmitter coil 12 acquired from the magnetic sensor 19.

Incidentally, in a second compression operation after the start of a series of compression operations, the second calculation section 233B calculates a compression depth Dr of the second compression operation based on a coil-to-coil distance AD and a correlation R of a first compression operation.

The compression depth calculating device 1000 determines excess or deficiency of the compression depth Dr which has been obtained in accordance with the average correlation Rave, and gives an instruction to the rescuer from the voice generating section 25. For example, when determination is made that the compression depth Dr is too small, voice guidance "Please press more strongly" is generated from the voice generating section 25. On the other hand, when determination is made that the compression depth Dr is too large, voice guidance "Please press more weakly" is generated by the voice generating section 25. Incidentally, the aforementioned voice message may be displayed by characters on the display section 26 in addition to or in place of the voice guidance.

As described above, according to the compression depth calculating device 1000 according to the embodiment, the coil-to-coil distance AD which is acquired from the magnetic sensor 19 is inputted to the average correlation Rave. The average correlation Rave is an average of the correlations R between the compression depth Da which has been calculated based on the second-order integration of the acceleration information acquired from the acceleration sensor 13 and the coil-to-coil distance AD which is the information corresponding to the magnitude of the compression of the compression operation which has been acquired from the magnetic sensor 19. With this configuration, since the information which is acquired from the magnetic sensor 19 is applied to the average correlation Rave, the compression depth Dr can be calculated accurately for every rescuer even when a sudden change in compression depth appears in one of compression operations. Although a lot of noise is contained in the second-order integrated value of the acceleration sensor 13, the influence of the noise can be reduced because the average correlation Rave which is the average of the correlations R is used. Thus, according to the aforementioned configuration, the compression depth Dr can be calculated accurately even when a compression displacement varies from one compression operation to another.

The compression depth calculating device 1000 can calculate the compression depth Dr accurately. Accordingly, the determination section 234 can determine excess or deficiency of the compression depth Dr accurately and give an accurate instruction to the rescuer from the voice generating section 25.

Incidentally, in the aforementioned embodiment, the average correlation Rave which is the average of the correlations R obtained by the third calculation section 233C is used when a compression depth Dr of a compression operation is calculated by the second calculation section 233B. However, the average correlation Rave does not have to be always used. For example, when the compression depth Dr of the compression operation is calculated by the second calculation section 233B, a correlation R obtained during a previous compression operation may be used.

Also in the case, the information which is acquired from the magnetic sensor 19 is applied to the correlation R. Accordingly, even when a sudden change in compression depth Dr appears in one of the compression operations, the compression depth Dr can be calculated accurately.

In addition, according to the embodiment, the magnetic sensor 19 is used as a sensor acquiring information corresponding to a magnitude of compression on a compressed part due to a compression operation. Accordingly, the coil-to-coil distance AD between the transmitter coil 12 and the receiver coil 11 can be obtained as the information corresponding to the magnitude of compression accurately from the magnetic sensor 19. The information of the coil-to-coil distance AD is applied to the aforementioned correlation Rave or the aforementioned correlation R. Accordingly, even when a sudden change in compression depth Dr appears in one of the compression operations, the compression depth Dr can be calculated accurately.

Incidentally, a correlation between the acceleration information acquired from the acceleration sensor 13 and second-order differentiation of the coil-to-coil distance AD acquired from the magnetic sensor 19 may be used as the correlation R to be used for calculation of the compression depth Dr.

The invention is defined by appended claims 1-3.

## Claims

1. A compression depth calculating device (1000) which is adapted to calculate a compression depth as a size of a recess of a compressed target, the device comprising:
a first sensor (13) adapted to detect acceleration of movement of a compressed part of the target due to a compression operation;
a second sensor (19) adapted to output information corresponding to a magnitude of compression on the compressed part of the target due to the compression operation;
a first calculation section (233A) adapted to calculate a correlation between a compression depth and the information corresponding to the magnitude of the compression by using (a) the compression depth calculated based on second-order integration of the acceleration information acquired from the first sensor and (b) the information corresponding to the magnitude of the compression acquired from the second sensor, the correlation between the compression depth and the information corresponding to the magnitude of the compression being modelled non-linearly ;
and
a second calculation section (233B) adapted to calculate a compression depth of a compression operation performed after the correlation has been calculated, based on the information corresponding to a magnitude of compression of the compression operation acquired from the second sensor and the correlation.

2. The compression depth calculating device according to Claim 1, further comprising:
a third calculation section (233C) adapted to calculate an average correlation which is an average of a plurality of the correlations calculated by the first calculation section; wherein:
the second calculation section (233B) is adapted to calculate a compression depth of a compression operation performed after the average correlation has been calculated, based on the information corresponding to a magnitude of compression of the compression operation acquired from the second sensor and the average correlation, when the compression operation is performed after the average correlation has been calculated.

3. The compression depth calculating device according to Claim 1 or Claim 2, wherein:
the second sensor (19) is a magnetic sensor;
the magnetic sensor includes:
a fixed section (15) that is fixed to the compressed part of the target;
a movable section (14) that is provided in an opposed position to the fixed section so as to be movable in a direction of the compression;
a magnetic field generating section (12) adapted to generate a magnetic field; and
a magnetic field detecting section (11) adapted to detect the magnetic field;
the magnetic field generating section (12) is provided in one of the fixed section (15) and the movable section (14), and the magnetic field detecting section (11) is provided in the other of the fixed section (15) and the movable section (14);
the first sensor (13) is disposed in the fixed section; and
the magnetic field generating section, the magnetic field detecting section, and the first sensor disposed in the fixed section are arranged side by side in the direction of the compression.

## Patentansprüche

1. Vorrichtung (1000) zur Berechnung der Kompressionstiefe, die angepasst ist, um eine Kompressionstiefe als eine Größe einer Aussparung eines komprimierten Ziels zu berechnen, wobei die Vorrichtung Folgendes umfasst:
einen ersten Sensor (13), der angepasst ist, die Beschleunigung der Bewegung eines komprimierten Teils des Ziels aufgrund eines Kompressionsvorgangs zu erfassen;
einen zweiten Sensor (19), der angepasst ist, Informationen auszugeben, die einer Größenordnung der Kompression des komprimierten Teils des Ziels aufgrund des Kompressionsvorgangs entsprechen;
einen ersten Berechnungsabschnitt (233A), der angepasst ist, um eine Korrelation zwischen einer Kompressionstiefe und den Informationen, die der Größenordnung der Kompression entsprechen, zu berechnen, indem (a) die Kompressionstiefe, die basierend auf der Integration zweiter Ordnung der von dem ersten Sensor erfassten Beschleunigungsinformationen berechnet wird, und (b) die Informationen, die der Größenordnung der Kompression entsprechen, die von dem zweiten Sensor erfasst werden, verwendet werden, wobei die Korrelation zwischen der Kompressionstiefe und den Informationen, die der Größenordnung der Kompression entsprechen, nichtlinear modelliert wird;
und
einen zweiten Berechnungsabschnitt (233B), der angepasst ist, um eine Kompressionstiefe eines Kompressionsvorgangs zu berechnen, der durchgeführt wird, nachdem die Korrelation berechnet worden ist, basierend auf den Informationen, die einer Größenordnung der Kompression des Kompressionsvorgangs entsprechen, die von dem zweiten Sensor und der Korrelation erfasst werden.

2. Vorrichtung zur Berechnung der Kompressionstiefe nach Anspruch 1, weiter umfassend:
einen dritten Berechnungsabschnitt (233C), der angepasst ist, eine durchschnittliche Korrelation zu berechnen, die ein Mittelwert aus einer Vielzahl von Korrelationen ist, die durch den ersten Berechnungsabschnitt berechnet wurden; wobei:
der zweite Berechnungsabschnitt (233B) angepasst ist, eine Kompressionstiefe eines Kompressionsvorgangs, der durchgeführt wird, nachdem die durchschnittliche Korrelation berechnet worden ist, basierend auf den Informationen, die einer Größenordnung der Kompression des Kompressionsvorgangs entsprechen, die von dem zweiten Sensor erfasst werden, und der durchschnittlichen Korrelation zu berechnen, wenn der Kompressionsvorgang durchgeführt wird, nachdem die durchschnittliche Korrelation berechnet worden ist.

3. Vorrichtung zur Berechnung der Kompressionstiefe nach Anspruch 1 oder Anspruch 2, wobei:
der zweite Sensor (19) ein Magnetsensor ist;
der Magnetsensor Folgendes einschließt:
einen befestigten Abschnitt (15), der an dem komprimierten Teil des Ziels befestigt ist;
einen beweglichen Abschnitt (14), der in einer dem befestigten Abschnitt gegenüberliegenden Position bereitgestellt ist, so dass er in einer Richtung der Kompression beweglich ist;
einen magnetfelderzeugenden Abschnitt (12), der angepasst ist, ein Magnetfeld zu erzeugen; und
einen magnetfelderkennenden Abschnitt (11), der angepasst ist, das Magnetfeld zu erkennen;
der magnetfelderzeugende Abschnitt (12) in einem von dem befestigten Abschnitt (15) und dem beweglichen Abschnitt (14) bereitgestellt ist und der magnetfelderkennende Abschnitt (11) in dem anderen von dem befestigten Abschnitt (15) und dem beweglichen Abschnitt (14) bereitgestellt ist;
der erste Sensor (13) in dem befestigten Abschnitt angeordnet ist; und
der magnetfelderzeugende Abschnitt, der magnetfelderkennende Abschnitt und der erste Sensor, der in dem befestigten Abschnitt angeordnet ist, in Richtung der Kompression nebeneinander angeordnet sind.

## Revendications

1. Dispositif de calcul de profondeur de compression (1000) qui est adapté pour calculer une profondeur de compression en tant que taille d'un évidement d'une cible compressée, le dispositif comprenant :
un premier capteur (13) adapté pour détecter une accélération de mouvement d'une partie compressée de la cible due à une opération de compression ;
un second capteur (19) adapté pour émettre des informations correspondant à une amplitude de compression sur la partie compressée de la cible due à l'opération de compression ;
une première section de calcul (233A) adaptée pour calculer une corrélation entre une profondeur de compression et les informations correspondant à l'amplitude de la compression en utilisant (a) la profondeur de compression calculée sur la base d'une intégration de second ordre des informations d'accélération obtenues à partir du premier capteur et (b) les informations correspondant à l'amplitude de la compression obtenues à partir du second capteur, la corrélation entre la profondeur de compression et les informations correspondant à l'amplitude de la compression étant modélisée de manière non linéaire ;
et
une deuxième section de calcul (233B) adaptée pour calculer une profondeur de compression d'une opération de compression réalisée après que la corrélation a été calculée, sur la base des informations correspondant à une amplitude de compression de l'opération de compression obtenues à partir du second capteur et de la corrélation.

2. Dispositif de calcul de profondeur de compression selon la revendication 1, comprenant en outre :
une troisième section de calcul (233C) adaptée pour calculer une corrélation moyenne qui est une moyenne d'une pluralité des corrélations calculées par la première section de calcul ; dans lequel :
la deuxième section de calcul (233B) est adaptée pour calculer une profondeur de compression d'une opération de compression réalisée après que la corrélation moyenne a été calculée, sur la base des informations correspondant à une amplitude de compression de l'opération de compression obtenues à partir du second capteur et de la corrélation moyenne, lorsque l'opération de compression est réalisée après que la corrélation moyenne a été calculée.

3. Dispositif de calcul de profondeur de compression selon la revendication 1 ou la revendication 2, dans lequel :
le second capteur (19) est un capteur magnétique ;
le capteur magnétique inclut :
une section fixe (15) qui est fixée à la partie compressée de la cible ;
une section mobile (14) qui est fournie dans une position opposée à la section fixe de manière à être mobile dans une direction de la compression ;
une section de génération de champ magnétique (12) adaptée pour générer un champ magnétique ; et
une section de détection de champ magnétique (11) adaptée pour détecter le champ magnétique ;
la section de génération de champ magnétique (12) est fournie dans l'une parmi la section fixe (15) et la section mobile (14), et la section de détection de champ magnétique (11) est fournie dans l'autre parmi la section fixe (15) et la section mobile (14) ;
le premier capteur (13) est disposé dans la section fixe ; et
la section de génération de champ magnétique, la section de détection de champ magnétique et le premier capteur disposé dans la section fixe sont agencés côte à côte dans la direction de la compression.
